# EUROPEAN PATENT APPLICATION

(11) **EP 4 480 569 A1**
(43) Date of publication of application: **25.12.2024**
(21) Application number: 23756360.6
(22) Date of filing: 14.02.2023
(51) Int. Cl.: B01D 61/58, C07C 29/152, C07C 31/04, C07B 61/00, B01D 53/22

(54) **REACTOR MODULE**

(30) Priority: 18.02.2022 JP 2022024238
(71) Applicant: NGK Insulators, Ltd., Nagoya-shi, Aichi 467-8530 (JP)
(72) Inventor: NAKAGAWA, Kosuke, Nagoya-shi, Aichi 467-8530 (JP); IIDA, Kazuki, Nagoya-shi, Aichi 467-8530 (JP); KAN, Hirofumi, Nagoya-shi, Aichi 467-8530 (JP); TORII, Atsushi, Nagoya-shi, Aichi 467-8530 (JP)
(74) Representative: Müller-Boré & Partner Patentanwälte PartG mbB
(86) International application number: PCT/JP2023/004983
(87) International publication number: WO 2023/157835

(57) **Abstract**

A reactor module (15) includes a pre-reactor (20) and a membrane reactor (30) disposed downstream of the pre-reactor (20) and including a separation membrane (34). An intermediate gas is generated from a source gas in the pre-reactor (20). The intermediate gas contains a liquid fuel, water vapor, and residual source gas. In the membrane reactor (30), the liquid fuel and water vapor are generated from the residual source gas. The separation membrane (34) allows the water vapor contained in the intermediate gas and the water vapor that is one of the products generated from the residual source gas to pass therethrough.

## Description

### TECHNICAL FIELD

The present invention relates to a reactor module.

### BACKGROUND ART

In recent years, membrane reactors capable of improving conversion efficiency by separating products in a conversion reaction for converting a source gas containing hydrogen and carbon oxide into a liquid fuel (methanol, ethanol, etc.) have been developed.

Patent Literature 1 discloses a membrane reactor including: a flow path through which a source gas flows; a catalyst disposed in the flow path; and a separation membrane that allows water vapor, which is one of products, to pass therethrough.

### CITATION LIST

### Patent Literature

Patent Literature 1: JP 2018-008940A

### SUMMARY

### TECHNICAL PROBLEM

In the membrane reactor described in Patent Literature 1, the amount of water vapor generated in an upstream region of the flow path is small, and the separation membrane cannot be fully utilized, so the cost performance of the separation membrane is low.

An object of the present invention is to provide a reactor module that can improve the cost performance of a separation membrane.

### SOLUTION TO PROBLEM

A reactor module according to the present invention includes a pre-reactor and a membrane reactor disposed downstream of the pre-reactor, the membrane reactor including a separation membrane. In the pre-reactor, an intermediate gas is generated from a source gas containing hydrogen and carbon oxide, the intermediate gas containing a liquid fuel, water vapor, and residual source gas. In the membrane reactor, the liquid fuel and water vapor are generated from the residual source gas. The separation membrane allows the water vapor contained in the intermediate gas and the water vapor generated from the residual source gas to pass therethrough.

### ADVANTAGEOUS EFFECTS

According to the present invention, it is possible to provide a reactor module that can improve the cost performance of a separation membrane.

### BRIEF DESCRIPTION OF DRAWINGS

FIG. 1 is a schematic diagram of a reactor module according to an embodiment.
FIG. 2 is a schematic diagram of a reactor module according to a variation.
FIG. 3 is a schematic diagram of a reactor module according to a variation.

### DESCRIPTION OF EMBODIMENTS

### Reactor System 100

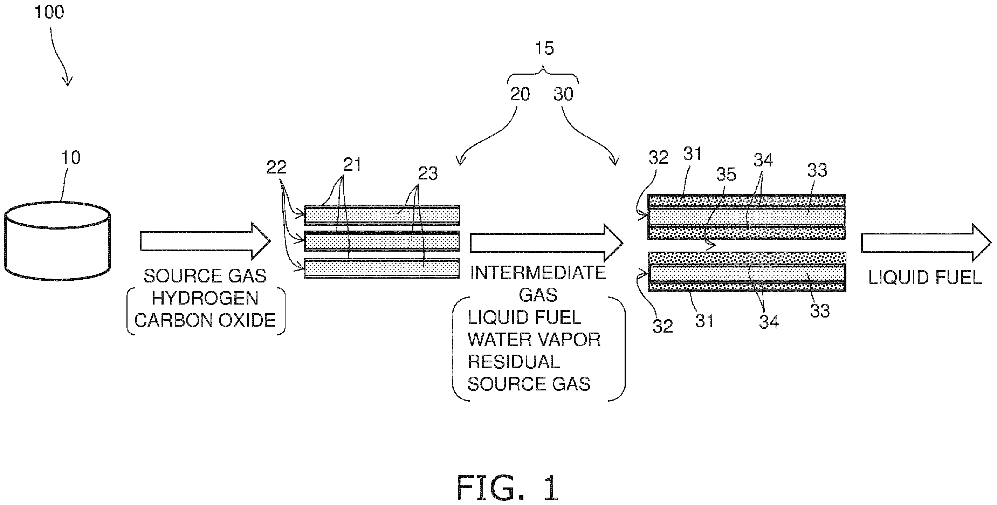

The following describes a reactor system 100 according to an embodiment. FIG. 1 is a schematic diagram showing the configuration of the reactor system 100. The reactor system 100 includes a source gas source 10 and a reactor module 15.

The reactor module 15 includes a pre-reactor 20 and a membrane reactor 30. The pre-reactor 20 and the membrane reactor 30 may be housed in separate pressure-resistant containers or may be housed together in a single pressure-resistant container. It is more preferable to house the pre-reactor 20 and the membrane reactor 30 in separate pressure-resistant containers, since temperatures of the pre-reactor 20 and the membrane reactor 30 can be controlled individually in such a case. FIG. 1 schematically shows cross sections of the pre-reactor 20 and the membrane reactor 30.

### Source Gas Source 10

The source gas source 10 is disposed upstream of the pre-reactor 20. A source gas is stored in the source gas source 10. The source gas source 10 supplies the source gas to the pre-reactor 20. The source gas contains at least hydrogen and carbon oxide. At least one of carbon monoxide and carbon dioxide can be used as the carbon oxide. The source gas may be a so-called synthesis gas (syngas).

### Pre-reactor 20

The pre-reactor 20 is disposed downstream of the source gas source 10. The pre-reactor 20 is disposed upstream of the membrane reactor 30. The source gas is supplied from the source gas source 10 to the pre-reactor 20. A conversion reaction for converting the source gas into a liquid fuel is carried out in the pre-reactor 20, and an intermediate gas is generated as a result of the conversion reaction. The pre-reactor 20 supplies the intermediate gas to the membrane reactor 30.

The liquid fuel is a fuel that is in a liquid state at normal temperature and normal pressure or a fuel that can be liquefied at normal temperature in a pressurized state. Examples of fuels that are in a liquid state at normal temperature and normal pressure include methanol, ethanol, a liquid fuel represented by CₙH₂₍ₘ₋₂ₙ₎ (m is an integer smaller than 90, and n is an integer smaller than 30), and a mixture of these. The liquid fuel generated in the pre-reactor 20 is in a gaseous state when it is generated, and remains in the gaseous state at least until the liquid fuel flows out from the pre-reactor 20. Examples of fuels that can be liquefied at normal temperature in a pressurized state include propane, butane, and a mixture of these.

For example, a reaction formula (1) for the synthesis of methanol through catalytic hydrogenation of a source gas containing hydrogen and carbon dioxide in the presence of a catalyst is expressed as follows.

CO₂+3H₂ ⇔ CH₃OH+H₂O (1)

The intermediate gas contains the liquid fuel generated as a result of the conversion reaction, water vapor, which is one of the products of the conversion reaction, and residual source gas that was not used in the conversion reaction. The content of water vapor in the intermediate gas can be determined depending on the permeability of a separation membrane 34 included in the membrane reactor 30, which will be described later. The content of the residual source gas in the intermediate gas can be determined depending on the conversion efficiency in the membrane reactor 30.

It is preferable that an operating temperature of the pre-reactor 20 is higher than an operating temperature of the membrane reactor 30. Since the pre-reactor 20 does not include a separation membrane, the operating temperature of the pre-reactor 20 can be set to a temperature suitable for the catalytic activity of a first catalyst 23 without consideration being given to the heat resistance of the separation membrane. The operating temperature of the pre-reactor 20 can be set to 180°C or higher and 350°C or lower, for example. The operating temperature of the pre-reactor 20 is the temperature of the intermediate gas flowing inside a portion adjacent to the pre-reactor 20 (hereinafter referred to as the "portion near the pre-reactor") in a connection pipe (not shown) connecting the pre-reactor 20 to the membrane reactor 30. The operating temperature of the pre-reactor 20 can be measured with use of a thermocouple, a resistance thermometer, a thermistor, or the like. When it is difficult to measure the temperature of the intermediate gas flowing inside the portion near the pre-reactor, it is also possible to set a measurement site on the outer surface of the portion near the pre-reactor, measure the temperature of the measurement site in a state where the influence of ambient temperature, etc., is reduced by covering the portion with a thermal insulation material, and estimate the temperature of the intermediate gas flowing inside the portion based on the measured temperature.

The pre-reactor 20 includes reaction tubes 21, first flow paths 22, and the first catalyst 23. The pre-reactor 20 does not include a separation membrane.

The first flow paths 22 are formed inside the reaction tubes 21. The source gas is caused to flow through the first flow paths 22. The first catalyst 23 is disposed in the first flow paths 22. The first catalyst 23 promotes the conversion reaction described above. The intermediate gas generated as a result of the conversion reaction is collected from the first flow paths 22. Although the pre-reactor 20 according to the present embodiment includes three first flow paths 22, it is sufficient that the pre-reactor 20 includes at least one first flow path 22.

A known catalyst suitable for the conversion reaction for obtaining the desired liquid fuel can be used as the first catalyst 23. Examples of the first catalyst 23 include metal catalysts (copper, palladium, etc.), oxide catalysts (zinc oxide, zirconia, gallium oxide, etc.), and composites of these (copper-zinc oxide, copper-zinc oxide-alumina, copper-zinc oxide-chromium oxide-alumina, copper-cobalt-titania, and these catalysts modified with palladium, etc.).

Although a configuration of the pre-reactor 20 has been described, the configuration of the pre-reactor 20 can be changed as necessary. It is possible to use, as the pre-reactor 20, a well-known reactor that does not include a separation membrane (for example, JP 2005-298413A or JP 2010-13422A).

### Membrane Reactor 30

The membrane reactor 30 is disposed downstream of the pre-reactor 20. The intermediate gas is supplied from the pre-reactor 20 to the membrane reactor 30. The membrane reactor 30 separates water vapor generated in the pre-reactor 20 and water vapor generated in the membrane reactor 30 while carrying out a conversion reaction for converting the residual source gas contained in the intermediate gas into the liquid fuel (see reaction formula (1) shown above). By carrying out the conversion reaction while separating a product as described above, it is possible to shift the reaction equilibrium of the above formula (1) to the product side by utilizing an equilibrium shift effect. Note that the liquid fuel generated in the membrane reactor 30 is in a gaseous state when it is generated, and remains in the gaseous state at least until the liquid fuel flows out from the membrane reactor 30.

The operating temperature of the membrane reactor 30 is set with consideration given to the heat resistance of the separation membrane 34. The operating temperature of the membrane reactor 30 may be lower than the operating temperature of the pre-reactor 20. The operating temperature of the membrane reactor 30 can be set to 160°C or higher and 300°C or lower, for example. The operating temperature of the membrane reactor 30 is the temperature of the liquid fuel flowing inside a portion adjacent to the membrane reactor 30 (hereinafter referred to as the "portion near the membrane reactor") in a discharge pipe (not shown) for discharging the liquid fuel to the outside from the membrane reactor 30. The operating temperature of the membrane reactor 30 can be measured with use of a thermocouple, a resistance thermometer, a thermistor, or the like. When it is difficult to measure the temperature of the liquid fuel flowing inside the portion near the membrane reactor, it is also possible to set a measurement site on the outer surface of the portion near the membrane reactor, measure the temperature of the measurement site in a state where the influence of ambient temperature, etc., is reduced by covering the portion with a thermal insulation material, and estimate the temperature of the liquid fuel flowing inside the portion based on the measured temperature.

The membrane reactor 30 includes a porous support 31, second flow paths 32, a second catalyst 33, the separation membrane 34, and a third flow path 35.

The porous support 31 is constituted by a porous material. A ceramic material, a metallic material, a resin material, or the like can be used as the porous material, and a ceramic material is particularly favorable. At least one of alumina (Al₂O₃), titania (TiO₂), mullite (Al₂O₃·SiO₂), potsherd, and cordierite (Mg₂Al₄Si₅O₁₈) can be used as an aggregate for the ceramic material, for example. At least one of titania, mullite, easily sinterable alumina, silica, glass frit, a clay mineral, and easily sinterable cordierite can be used as an inorganic binder for the ceramic material, for example. However, the ceramic material does not necessarily need to contain an inorganic binder.

The second flow paths 32 are formed in the porous support 31. The second flow paths 32 extend through the porous support 31. Accordingly, both ends of each second flow path 32 are open in the outer surface of the porous support 31. The second flow paths 32 correspond to a space on a non-permeation side of the separation membrane 34.

The intermediate gas is caused to flow through the second flow paths 32. The intermediate gas contains the liquid fuel generated in the pre-reactor 20, water vapor generated in the pre-reactor 20, and residual source gas that was not used in the conversion reaction in the pre-reactor 20. The membrane reactor 30 according to the present embodiment includes two second flow paths 32, but it is sufficient that the membrane reactor 30 includes at least one second flow path 32.

The second catalyst 33 is disposed in the second flow paths 32. A known catalyst suitable for the conversion reaction for obtaining the desired liquid fuel can be used as the second catalyst 33. The second catalyst 33 promotes the conversion reaction. Examples of the second catalyst 33 include metal catalysts (copper, palladium, etc.), oxide catalysts (zinc oxide, zirconia, gallium oxide, etc.), and composites of these (copper-zinc oxide, copper-zinc oxide-alumina, copper-zinc oxide-chromium oxide-alumina, copper-cobalt-titania, and these catalysts modified with palladium, etc.).

Here, the second catalyst 33 is used for the conversion reaction for converting the residual source gas contained in the intermediate gas. Therefore, the burden on the second catalyst 33 can be reduced when compared with a case where the second catalyst 33 is directly used for the conversion reaction for converting the whole amount of source gas. As described above, when the first catalyst 23 included in the pre-reactor 20 takes on a part of the burden required for the conversion reaction for converting the source gas, it is possible to reduce the burden on the second catalyst 33 included in the membrane reactor 30, thereby extending the service life of the second catalyst 33.

The separation membrane 34 is supported by the porous support 31. The separation membrane 34 surrounds the second flow paths 32. The separation membrane 34 is disposed between the second flow paths 32 and the third flow path 35.

The separation membrane 34 allows water vapor to pass therethrough. Specifically, the separation membrane 34 allows water vapor originally contained in the intermediate gas and water vapor newly generated from the residual source gas contained in the intermediate gas to pass therethrough.

Here, the amount of water vapor newly generated from the residual source gas is relatively small in an upstream region of the second flow paths 32, compared with a downstream region of the second flow paths 32. Therefore, an upstream portion of the separation membrane 34 is mainly used for permeation of water vapor originally contained in the intermediate gas, and a downstream portion of the separation membrane 34 is mainly used for permeation of water vapor newly generated from the residual source gas. As described above, it is possible to utilize the upstream portion of the separation membrane 34 by carrying out the conversion reaction with use of the intermediate gas containing water vapor, so the entire separation membrane 34 can be utilized effectively. Therefore, the cost performance of the separation membrane 34 can be improved.

An inorganic membrane can be used as the separation membrane 34. Inorganic membranes have heat resistance, pressure resistance, and water vapor resistance and thus are preferable. Examples of inorganic membranes include zeolite membranes, silica membranes, alumina membranes, and composite membranes thereof. In particular, LTA-type zeolite membranes in which a molar ratio (Si/Al) between silicon element (Si) and aluminum element (Al) is 1.0 or more and 3.0 or less have excellent water vapor permeability and thus are favorable.

It is preferable that the separation membrane 34 has a water vapor permeability coefficient of 100 nmol/(s·Pa·m²) or more. The water vapor permeability coefficient can be obtained using a known method (see Ind. Eng. Chem. Res., 40, 163-175 (2001)).

It is preferable that the separation membrane 34 has a separation coefficient of 100 or more. The greater the separation coefficient, the easier it is for water vapor to pass through the separation membrane 34 and the more difficult it is for components (hydrogen, carbon dioxide, the liquid fuel, etc.) other than water vapor to pass through the separation membrane 34. The separation coefficient can be obtained using a known method (see FIG. 1 in "Separation and Purification Technology 239 (2020) 116533").

The third flow path 35 is formed in the porous support 31. The third flow path 35 extends through the porous support 31. Accordingly, both ends of the third flow path 35 are open. The second flow path 32 corresponds to a space on a permeation side of the separation membrane 34.

A sweep gas for sweeping water vapor that has passed through the separation membrane 34 is caused to flow through the third flow path 35. Inert gas (e.g., nitrogen), air, or the like can be used as the sweep gas. Although the membrane reactor 30 according to the present embodiment includes a single third flow path 35, it is sufficient that the membrane reactor 30 includes at least one third flow path 35. No catalyst is disposed in the third flow path 35.

Although a configuration of the membrane reactor 30 has been described above, the configuration of the membrane reactor 30 can be changed as necessary. It is possible to use, as the membrane reactor 30, a well-known reactor including a separation membrane, such as a tube type reactor (for example, JP 2018-008940A) or a monolith type reactor. The term "monolith" means a structure that includes a plurality of holes extending through the reactor in the longitudinal direction, and encompasses a honeycomb structure.

### Variations of Embodiment

Although an embodiment of the present invention has been described, the present invention is not limited to the above embodiment, and various changes can be made within a scope not departing from the gist of the present invention.

### Variation 1

In the above embodiment, the reactor module 15 includes only one membrane reactor 30, but the reactor module 15 may include a plurality of membrane reactors 30.

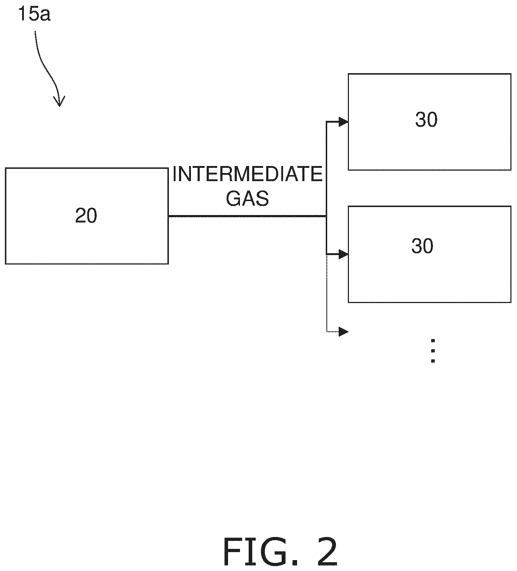

For example, a plurality of membrane reactors 30 can be arranged in parallel as in a reactor module 15a shown in FIG. 2. In this case, each membrane reactor 30 is connected to the pre-reactor 20. The intermediate gas generated in the pre-reactor 20 is distributed to each membrane reactor 30.

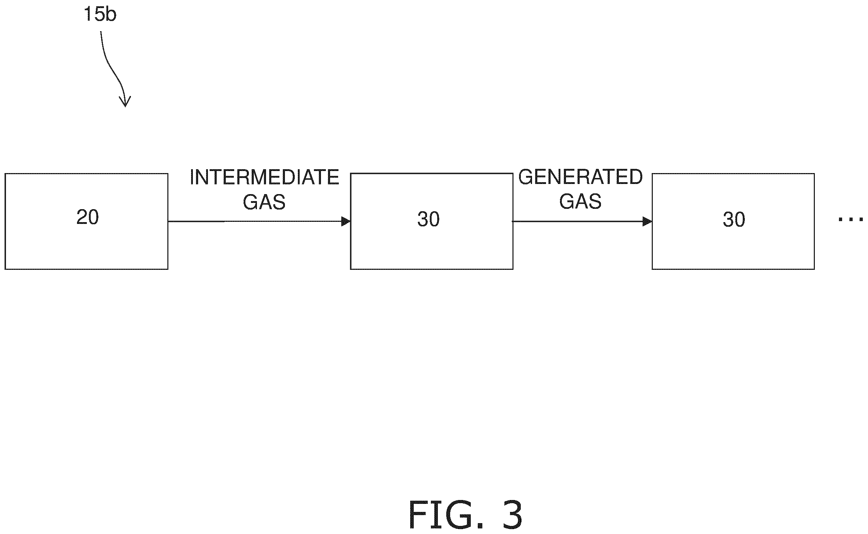

Alternatively, a plurality of membrane reactors 30 can be arranged in series as shown in a reactor module 15b shown in FIG. 3. In this case, the most upstream membrane reactor 30 of the plurality of membrane reactors 30 is connected to the pre-reactor 20, and the remaining membrane reactors 30 are connected downstream of the most upstream membrane reactor 30. The intermediate gas generated in the pre-reactor 20 is supplied to the most upstream membrane reactor 30. Gas generated in each membrane reactor 30 is supplied to the other membrane reactors 30 located downstream. The generated gas contains the liquid fuel and residual source gas. The generated gas may contain water vapor.

Alternatively, a plurality of membrane reactors 30 may be arranged in parallel and in series, although this arrangement is not shown.

As described above, when a plurality of membrane reactors 30 are disposed downstream of the pre-reactor 20, it is possible to reduce the burden on the second catalyst 33 included in each of the membrane reactors 30 by adopting a configuration in which the first catalyst 23 included in the pre-reactor 20 takes on a part of the burden required for the conversion reaction for converting the source gas.

### Variation 2

In the above embodiment, the sweep gas is caused to flow through the third flow path 35, but the sweep gas does not necessarily need to be caused to flow through the third flow path 35.

### Variation 3

In the above embodiment, the intermediate gas is supplied directly from the pre-reactor 20 to the membrane reactor 30, but the intermediate gas may also be supplied to the membrane reactor 30 after being cooled. For example, a cooling device (a radiator or a heat exchanger) may be disposed between the pre-reactor 20 and the membrane reactor 30 to cool the intermediate gas in the cooling device.

### Variation 4

In the above embodiment, the separation membrane 34 allows water vapor generated in the pre-reactor 20 and water vapor generated in the membrane reactor 30 to pass therethrough, but there is no limitation to this configuration. The separation membrane 34 may allow the liquid fuel generated in the pre-reactor 20 and the liquid fuel generated in the membrane reactor 30 to pass therethrough. In this case as well, it is possible to shift the reaction equilibrium of the above formula (1) to the product side.

Also, in the case where the separation membrane 34 allows the liquid fuel to pass therethrough, the reaction equilibrium can be shifted to the product side even when the liquid fuel is generated as a result of a reaction in which no water vapor is generated (e.g., 2H₂+CO ⇔ CH₃OH).

### Variation 5

In the above embodiment, the source gas containing hydrogen and carbon oxide is supplied directly to the pre-reactor 20, but a configuration is also possible in which the source gas is generated in the pre-reactor 20, and at the same time, the liquid fuel is generated with use of the generated source gas.

For example, in a case where ammonia and carbon dioxide are supplied to the pre-reactor 20, hydrogen, which is a component of the source gas, is generated from the ammonia in accordance with the following reaction formula (2), and methanol is generated from the source gas (hydrogen and carbon dioxide) in accordance with the reaction formula (3). Note that the reaction formula (4) is obtained by combining the reaction formulas (2) and (3).

NH₃ → 1/2N₂O+3/2H₂ (2)

CO₂+4H₂ → CH₄+2H₂O (3)

CO₂+8/3NH₃ → CH₄+4/3N₂+2H₂O (4)

### REFERENCE SIGNS LIST

- 10: Source gas source
- 15: Reactor module
- 20: Pre-reactor
- 21: Reaction tube
- 22: First flow path
- 23: First catalyst
- 30: Membrane reactor
- 31: Porous support
- 32: Second flow path
- 33: Second catalyst
- 34: Separation membrane
- 35: Third flow path

## Claims

1. A reactor module comprising:
a pre-reactor; and
a membrane reactor disposed downstream of the pre-reactor, the membrane reactor including a separation membrane, wherein
in the pre-reactor, an intermediate gas is generated from a source gas containing hydrogen and carbon oxide, the intermediate gas containing a liquid fuel, water vapor, and residual source gas,
in the membrane reactor, the liquid fuel and water vapor are generated from the residual source gas, and
the separation membrane allows the water vapor contained in the intermediate gas and a product generated from the residual source gas to pass therethrough.

2. The reactor module according to claim 1, wherein
an operating temperature of the pre-reactor is higher than an operating temperature of the membrane reactor.

3. The reactor module according to claim 1 or 2, wherein
the pre-reactor includes a first flow path through which the source gas flows and a first catalyst disposed in the first flow path, and
the membrane reactor includes a second flow path through which the intermediate gas flows and a second catalyst disposed in the second flow path.

4. The reactor module according to claim 1 or 2, comprising:
a plurality of the membrane reactors, wherein
the plurality of membrane reactors are arranged in parallel.

5. The reactor module according to claim 1 or 2, comprising:
a plurality of the membrane reactors, wherein
the plurality of membrane reactors are arranged in series.
